(19) Europäisches Patentamt
European Patent Office
Office européen des brevets

(11) Numéro de publication: **0 318 377 B1**

(12) **FASCICULE DE BREVET EUROPEEN**

(45) Date de publication de fascicule du brevet: **25.08.93**

(51) Int. Cl.[5]: **C07C 327/22**, C07C 327/34, A61K 31/135

(21) Numéro de dépôt: **88402944.8**

(22) Date de dépôt: **24.11.88**

Le dossier contient des informations techniques présentées postérieurement au dépôt de la demande et ne figurant pas dans le présent fascicule.

# (54) Nouveaux composés énantiomères dérivés d'amino-acides, leur procédé de préparation et leurs applications thérapeutiques.

(30) Priorité: **24.11.87 FR 8716239**

(43) Date de publication de la demande: **31.05.89 Bulletin 89/22**

(45) Mention de la délivrance du brevet: **25.08.93 Bulletin 93/34**

(84) Etats contractants désignés: **AT BE CH DE ES FR GB GR IT LI LU NL SE**

(56) Documents cités:
**EP-A- 0 038 758**
**FR-A- 2 559 770**

**THE JOURNAL OF PHARMACOLOGY AND EXPERIMENTAL THERAPEUTICS, vol. 243, no. 2, 1987, pages 666-673, The American Society for Pharmacology and Experimental Therapeutics, US; B. GIROS et al.: "Enantiomers of thiorphan and acetorphan: Correlation between enkephalinase inhibition, protection of endogenous enkephalins and behavioral effects"**

(73) Titulaire: **SOCIETE CIVILE BIOPROJET**
**30, rue des Francs Bourgeois**
**F-75003 Paris(FR)**

(72) Inventeur: **Duhamel, Pierre**
**32 rue Jacques-Boutrolle**
**F-76130 Mont Saint Aignan(FR)**
Inventeur: **Duhamel, Lucette**
**32 rue Jacques-Boutrolle**
**F-76130 Mont Saint Aignan(FR)**
Inventeur: **Danvy, Denis**
**Bûtot Venesville**
**F-76450 Cany Barville(FR)**
Inventeur: **Plaquevent, Jean-Christophe**
**74 allée Messager**
**F-76690 Notre Dame de Bondeville(FR)**
Inventeur: **Giros, Bruno**
**3 allée Lech Walesa**
**F-94270 Kremlin Bicetre(FR)**
Inventeur: **Gros, Claude**
**31 rue Robert de Flers**
**F-75015 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 318 377 B1

Rank Xerox (UK) Business Services
(3.10/3.6/3.3.1)

**THE JOURNAL OF ORGANIC CHEMISTRY, vol. 50, no. 11, 31 mai 1985, pages 1830-1835, American Chemical Society, Washington, DC, US; D.A. EVANS et al.: "Asymmetric synthesis of the enkephalinase inhibitor thiorphan"**

Inventeur: **Schwartz, Jean-Charles**
**9 Villa Seurat**
**F-75014 Paris(FR)**
Inventeur: **Lecomte, Jeanne-Marie**
**30 rue des Francs Bourgeois**
**F-75003 Paris(FR)**

(74) Mandataire: **Bernasconi, Jean et al**
**CABINET LEMOINE ET BERNASCONI 13, Boulevard des Batignolles**
**F-75008 Paris (FR)**

## Description

La présente invention est relative à de nouveaux composés énantiomères dérivés d'amino-acides, à leur procédé de préparation et à leurs applications thérapeutiques.

Le composé N-((mercaptométhyl)-2 oxo-1 phényl-3 propyl)- glycine ou thiorphan a été décrit sous sa forme racémique (R, S) comme un puissant inhibiteur de l'enképhalinase, enzyme responsable de l'inactivation des enképhalines, et comme un moins puissant inhibiteur de l'enzyme de conversion de l'angiotensine ou ACE (Roques et al., Nature, 1980, 288, 286, Roques et al. EP-A-0038758; US-A-4513009). Ce composé présente des propriétés analgésiques par voie intraveineuse et intracérébroventriculaire liées à la protection des enképhalines endogènes mais aussi, à dose plus élevée, une activité cardiovasculaire qui peut, soit présenter des inconvénients, soit être recherchée pour certaines applications thérapeutiques. Ces mêmes propriétés et inconvénient potentiel se retrouvent dans un de ses dérivés, l'acétorphan ou ester benzylique de N-((acéthylthiométhyl)-2 oxo-1 phényl-3 propyl)glycine (R, S) qui présente cependant l'avantage d'être actif par voie intraveineuse, à faible dose (voisine de 1 mg/kg) alors que le (R, S) thiorphan n'est actif que par voie intracérébrale ou à des doses environ 50 fois plus élevées, par voie générale (Lecomte et al., J. Pharmacol. Exp. Ther., 1986, 237, 937).

Récemment, un procédé de synthèse asymétrique, difficilement applicable à la préparation industrielle, a permis d'obtenir séparément les deux énantiomères du thiorphan (Evans et al., J. Org. Chem. 1985, 50, 1830) et de montrer que, alors que ces deux composés présentent une activité inhibitrice de l'enképhalinase voisine, l'activité inhibitrice de l'ACE résidait principalement dans l'isomère (S) et l'activité analgésique (par voie intracérébrale) principalement dans l'isomère (R). Par ailleurs, l'équi-activité, vis-à-vis de l'enképhalinase des deux isomères du thiorphan, obtenus ici encore par un procédé de séparation difficilement applicable à une préparation industrielle, a été confirmée (Fournié-Zaluski et al. J. Med. Chem. 1986, 29, 751).

Le but de la présente invention est :

1. de fournir, à titre de produits industriels nouveaux, des préparations sensiblement pures des isomères (R) et (S) de l'acétorphan et de dérivés voisins,
2. d'apporter un nouveau procédé de séparation applicable à la préparation industrielle des isomères (R) et (S) de l'acétorphan et de dérivés voisins,
3. de décrire les activités analgésiques observées après administration à dose élevée par voie orale et sans effets secondaires gênants liés à l'inhibition de l'ACE,
4. de décrire un certain nombre d'autres effets de ces composés sur le tube digestif dont l'ensemble autorise une activité thérapeutique originale dans un certain nombre de troubles fonctionnels tels que le syndrome du "côlon irritable" pour lequel aucun traitement efficace n'a été proposé, ainsi qu'un effet protecteur du facteur auriculaire natriurétique qui autorise une activité thérapeutique originale dans les domaines cardiovasculaire et rénal tels que l'insuffisance cardiaque, l'hypertension artérielle et l'insuffisance hépatorénale.
5. de fournir, par l'intermédiaire thérapeutique de ces composés, de nouveaux médicaments.

Les nouveaux dérivés de l'invention répondent à la formule :

$$CH_3 - CO - S - CH_2 - \underset{\displaystyle CH_2 - C_6H_5}{\underset{|}{CH}} - CO - NH - CH_2 - COO\ R1$$

(R) et (S)

dans laquelle

R1 = $CH_3$ ou $CH_2 - C_6H_5$

Les composés ainsi définis sont les suivants :

Composé (1)

$CH_3 - CO - S - CH_2 - CH - CO - NH - CH_2 - COO\ CH_2 - C_6H_5$

$\qquad\qquad\qquad\qquad\quad | $

$\qquad\qquad\qquad\qquad CH_2 - C_6H_5 \qquad (R)$

Composé (2)

$CH_3 - CO - S - CH_2 - CH - CO - NH - CH_2 - COO\ CH_2 - C_6H_5$

$\qquad\qquad\qquad\qquad\quad | $

$\qquad\qquad\qquad\qquad CH_2 - C_6H_5 \qquad (S)$

Composé (3)

$CH_3 - CO - S - CH_2 - CH - CO - NH - CH_2 - COO - CH_3$

$\qquad\qquad\qquad\qquad\quad | $

$\qquad\qquad\qquad\qquad CH_2 - C_6H_5 \qquad (R)$

Composé (4)

$CH_3 - CO - S - CH_2 - CH - CO - NH - CH_2 - COO - CH_3$

$\qquad\qquad\qquad\qquad\quad | $

$\qquad\qquad\qquad\qquad CH_2 - C_6H_5 \qquad (S)$

L'invention concerne également un médicament caractérisé en ce qu'il comporte, comme principe actif, l'un des quatre nouveaux composés précités.

L'invention concerne également l'utilisation des nouveaux composés précités, ainsi que des composés (5) et (6) suivants pour la préparation de nouveaux médicaments, les composés (1), (3) et (5), (R) étant utilisés pour la préparation de médicaments du syndrome du colon irritable, de médicaments du reflux gastro-oesophagien et de médicaments de la cholécystite aiguë, alors que les composés (2), (4) et (6), (S) sont utilisés pour la préparation de médicaments pour le traitement de l'hypertension artérielle et de médicaments pour le traitement de l'insuffisance cardiaque et hépatorénale.

Composé (5)

$CH_3 - CO - S - CH_2 - CH - CO - NH - CH - COO - CH_3$

$\qquad\qquad\qquad\qquad\quad | \qquad\qquad\qquad\quad |$

$\qquad\qquad\qquad\qquad CH_2 - C_6H_5 \qquad CH_3 \qquad\qquad (R),(S)$

Composé (6)

$CH_3 - CO - S - CH_2 - CH - CO - NH - CH - COO - CH_3$

$\qquad\qquad\qquad\qquad\quad | \qquad\qquad\qquad\quad |$

$\qquad\qquad\qquad\qquad CH_2C_6H_5 \qquad\quad CH_3$

$(S),(S)$

Les composés de la présente invention sont préparés par la suite de réactions ci-dessous.

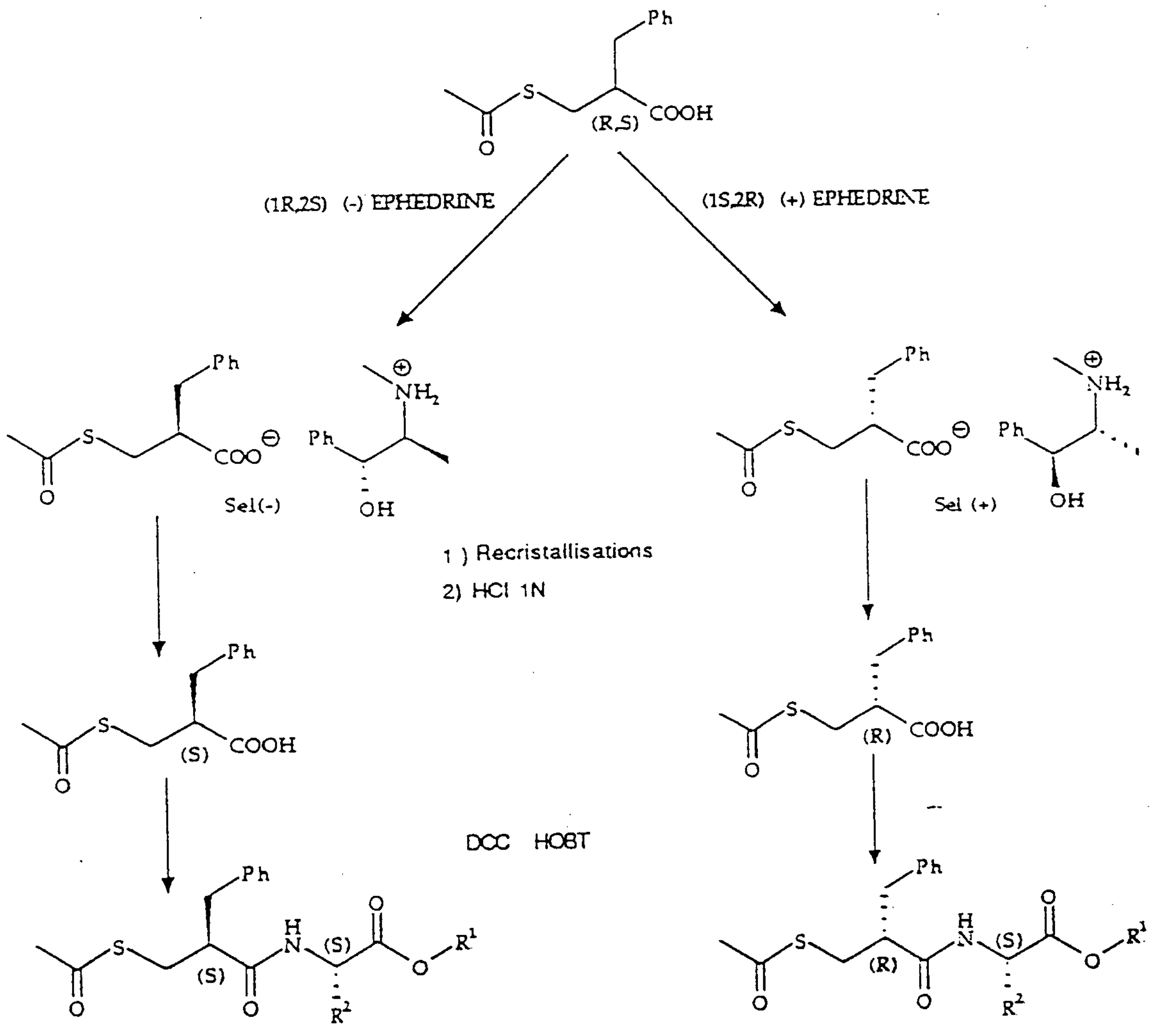


| | | | |
|---|---|---|---|
| Ex.2 : R1 = CH2Ph | R2 = H | Ex.1 : R1 = CH2Ph | R2 = H |
| Ex.4 : R1 = CH3 | R2 = H | Ex.3 : R1 = CH3 | R2 = H |
| Ex.6 : R1 = CH3 | R2 = CH3 | Ex.5 : R1 = CH3 | R2 = CH3 |

L'étape initiale est le dédoublement de l'acide acétylthio-3 benzyl 2-propanoïque. Pour ce faire, on fait réagir l'acide racémique de préférence avec l'éphédrine (+), ou selon le cas (-) notamment dans l'éther. Le sel obtenu est recristallisé, par exemple dans un mélange de dichlorométhane/éther de pétrole. Les sels obtenus sont optiquement purs. L'acide est libéré par action, par exemple, d'acide chlorhydrique et le couplage avec l'ester correspondant de l'acide aminé (glycine ou alanine) est réalisé, de préférence, en présence de dicyclohexyl carbodiimide (DCC) comme agent de couplage et d'hydroxybenzotriazole (HOBT) pour éviter la racémisation.

Exemple 1

Ester benzylique de la N- ((acétylthiométhyl)-2 oxo-1 phényl-3 propyl)-glycine (R).

Synthèse d'acétylthio-3-benzyl 2-propionate d'éphédrinium (+).

Dans un erlenmeyer, on introduit 10 g d'acide acétylthio-3 benzyl-2 propanoïque (42 mmol) racémique. On le dissout dans 50 ml d'éther, puis on ajoute 3,47 g de (+) éphédrine (21 mmol). On agite jusqu'à dissolution et laisse cristalliser à température ambiante. Les premiers cristaux apparaissent au bout de

quelques heures (3h). On filtre le sel après 3 jours de repos à température ambiante. Le sel est broyé et lavé par 5 ml d'éther.

Masse de sel (+) obtenu : 4,2 g - Rendement : 49 %

F = 103 - 114°C (Microscope)

$(\alpha)_D^{25}$ = +43,0° (c = 1,20 dans le MeOH)

On dissout 4,1 g de sel ( $(\alpha)_D^{25}$ = +43,0°) dans 20 ml de dichlorométhane chaud. On revient à température ambiante puis on ajoute 20 ml d' éther de pétrole. On laisse cristalliser à température ambiante. On filtre après 33 heures.

Masse de sel (+) obtenu : 2,80 g - Rendement : 68 %.

F = 115 - 123°C (Microscope)

$(\alpha)_D^{25}$ = +47,5° (c = 1,20 dans le MeOH)

On répète l'opération précédente en dissolvant 2,39 g de sel ($(\alpha)_D^{25}$ = + 47,5°) dans 11 ml de CH2Cl2 chaud et 7 ml d'éther de pétrole. On laisse cristalliser pendant 23 heures. On filtre.

Masse de sel obtenu : 1,69 g - Rendement : 71 %.

Rendement des 2 recristallisations : 48 %.

F = 122 -124°C (Microscope)

$(\alpha)_D^{25}$ = +49,2° (c = 1,20 dans le MeOH).

Synthèse de l'acide R (+) acétylthio-3 benzyl-2 propanoïque.

On met en solution 1,6 g du sel précèdent (+) ($(\alpha)_D^{25}$ = +49,2°) dans un mélange de 20 ml d'eau et de 15 ml de CH2Cl2. On ajoute de l'acide chlorhydrique 1N jusqu'à pH = 1. On sépare la phase organique et on extrait la phase aqueuse par 15 ml de CH2Cl2. Les phases organiques sont réunies, séchées sur MgSO4 et concentrées pour donner l'acide (+) acétylthio-3 benzyl-2 propanoïque.

Masse d'acide (+) obtenu : 0,95 g - rendement : 97 %.

$(\alpha)_D^{25}$ = +35,3° (c = 1,30 dans le MeOH).

Dans un tricol muni d'une garde à chlorure de calcium et d'une agitation magnétique, on place 3,74 g d'acide R (+) (15,7 mmol, $(\alpha)_D^{25}$ = +35,4°) en solution dans 26 ml de THF anhydre. On ajoute successivement, à environ 0-5°C, 5,29 g de glycinate de benzyle (sous forme de paratoluène sulfonate, 15,7 mmol) et 1,58 g de triéthylamine (15,7 mmol) dans 30 ml de chloroforme, 2,40 g d'hydroxybenzotriazole (HOBT) (15,7 mmol) dans 22 ml de THF et 3,24 g de dicyclohexylcarbodiimide (DCC) (15,7 mmol) dans 19 ml de chloroforme. On laisse la température revenir à 20°C, puis on agite pendant 5 h.

La dicyclohexylurée (DCU) est filtrée et on évapore à sec. Le résidu jaune est repris par 40 ml d'acétate d'éthyle. La DCU qui a, à nouveau, précipité est filtrée. La phase organique est successivement lavée par 1 fois 20 ml d'eau, 3 fois 20 ml d'une solution saturée d'hydrogénocarbonate de sodium, 1 fois 20 ml d'eau et 1 fois 20 ml d'une solution saturée de chlorure de sodium. On sèche sur sulfate de magnésium et on évapore à sec. On obtient un résidu solide jaune. On le dissout dans 25 ml d'éther à ébullition. On laisse refroidir. On filtre et sèche sous vide sur pentaoxyde de phosphore. On obtient un solide blanc.

Masse du composé 1 R (+) : 5,31 g - Rendement 87 %.

F : 69°C (K) (Kofler)

$(\alpha)_D^{25}$ = +23,9° (c = 1,2 MeOH)

RMN 1H ; 7,36 (s,5H) ; 7,24 (s,5H) ; 5,85 (s, 1H) ; 5,14 (s, 2H) ; 3,95 (dd, 2H, J1 = 3,3 Hz, J2 = 4,6 Hz) ; 3,22 à 2,74 (m, 5H) ; 2,34 (s, 3H) (CDCl3, TMS).

IR (cm -1) : 3280 ; 1755 ; 1695 ; 1640 (Nujol).

RMN 13 C : 195,8 (s) ; 172,9 (s) ; 169,2 (s) ; 138,4 (s) ; 134,9 (s) ; 128,7 (d) ; 128,4 (d) ; 128,2 (d) ; 127,7 (d) ; 127,5 (d) ; 127,3 (d) ; 126,5 (d) ; 66,9 (t) ; 49,1 (d) ; 41,2 (t) ; 38,2 (t) ; 31,0 (t) ; 30,4 (q) (CDCl3).

Ce composé, R(+), sera désigné, ci-après, composé(1).

| Microanalyse : | | | | | | |
|---|---|---|---|---|---|---|
| Trouvé<br>Calculé | C% : | 65,34 ;<br>65,45 | N% : | 3,95 ;<br>3,63 | H% : | 6,10<br>5,97. |

Exemple 2 : Ester- benzylique de la N-((acétylthiométhyl)-2 oxo-1 phényl-3 propyl)- glycine (S) (-).

On suit le mode opératoire décrit dans l'exemple 1 en utilisant la (-) éphédrine. Les caractéristiques de l'acétylthio-3 benzyl-2 propionate d'éphédrinium (-) sont identiques à celles de son énantiomère décrit dans l'exemple 1, à l'exception du pouvoir rotatoire

F = 124 -126°C (Microscope)

$(\alpha)_D^{25}$ = -49,3° (1,4 MeOH).

L'acide S (-) acétylthio 3-benzyl-2 propanoïque est libéré dans les mêmes conditions que dans l'exemple 1

$(\alpha)_D^{25}$ = -36,4° (1,3 MeOH).

Pour la synthèse du (S) acétorphan, on suit le mode opératoire décrit dans l'exemple 1, à partir de l'acide S (-).

Rendement : 84 %.

$(\alpha)_D^{25}$ = -24,1°, c=1,3, MeOH.

Les caractéristiques spectrales de l'exemple 2 S (-) sont identiques à celles de son énantiomère.

F : 71°C (K).

| Microanalyse: | | | | | | |
|---|---|---|---|---|---|---|
| Trouvé<br>Calculé | C% : | 65,02 ;<br>65,45 | N% : | 3,88 ;<br>3,63 | H% | 5,87<br>5,97. |

Ce composé sera désigné, ci-après, composé (2).

Dans une variante, on peut également préparer le composé (2), à titre d'exemple, en partant du filtrat obtenu dans l'exemple 1 après extraction de l'acétylthio-3 benzyl 2-propionate d'éphédrinium (+). On traite le filtrat par la (-) éphédrine, le traitement se poursuivant comme dans l'exemple 2.

Exemple 3.

Ester méthylique de la N-((acétylthiométhyl)-2 oxo-1 phényl-3 propyl)- glycine (R).

Dans les conditions décrites pour l'exemple 1, l'acide R (+) est couplé avec le glycinate de méthyle et le produit attendu est obtenu avec un rendement comparable. Ce composé sera désigné, ci-après, composé (3).

rendement : 84 % (chromatographie sur gel de silice)

F = 76-77°C (Kofler)

$(\alpha)_D^{25}$ = + 29,6° (c = 1,0 ; MeOH).

IR(nujol) ($cm^{-1}$) : 3300, 1755, 1690, 1660

RMN$^1$H (CDCl3) : 7,15 (S,5H) ; 6,25 (t, 1H, J = 5,6 Hz) ; 3,9 (dd, 1H, J = 5,6 Hz) ; 3,85 (dd, 1H, J = 5,6 Hz) ; 3,6 (s, 3H) ; 3,2 à 2,5 (m, 5H), 2,2 (s, 3H).

RMN$^{13}$C (CDC13) : 196,0(s) ; 172,9 (s) ; 169,7 (s) ; 138,3 (s) ; 128,6 (d) ; 128,2 (d) ; 126,3 (d) ; 51,9 (q) ; 48,6 (d) ; 40,8 (t) ; 38,0 (t) ; 30,8 (t) ; 30,3 (Q)

| Microanalyse : | | | | | | |
|---|---|---|---|---|---|---|
| trouvé<br>calculé | C % : | 58,32 ;<br>58,25 | N % : | 4,54 ;<br>4,53 | H % | 6,14<br>6,14. |

Exemple 4.

Ester méthylique de la N-((acétylthiométhyl)-2 oxo-1 phényl-3 propyl)-glycine (S). L'acide S (-) est couplé, dans les conditions décrites pour l'obtention de l'exemple 2, avec le glycinate de méthyle. Ce composé sera désigné, ci-après, composé (4).

Exemple 5.

Ester méthylique de la (R)N((acétylthiométhyl)-2 oxo-1 phényl-3 propyl)-(S) alanine :

L'acide R(+) est couplé dans les conditions décrites pour l'obtention de l'exemple 1, avec le (S) alaninate de méthyle. Ce composé sera désigné, ci-après, composé (5).

Rendement : 70 %

F : 56°C (Microscope)

$(\alpha)_D^{25}$ = + 9,1° (1,37, MeOH)

IR : 3300, 1735, 1680, 1650 (nujol)

RMN[1]H (CDCl3) : 7,2 (s, 5H) ; 6,6 (m, 1H) ; 4,5 (m, 1H) ; 2,7 à 3,3 (m, 5H) ; 2,3 (s, 3H) ; 1,1 (d, J = 7,3 Hz, 3H).

RMN[13]C (CDCl3) : 195,6 (s) ; 172,8 (s) ; 171,9 (s), 138,3 (s) ; 128,6 (d) ; 128,1 (d) ; 126,2 (d) ; 52,0 (q) ; 49,0 (d) ; 47,4 (d) ; 38,3 (t) ; 30,9 (t) ; 30,2 (q) ; 17,8 (q)

| Microanalyse : | | | | | | |
|---|---|---|---|---|---|---|
| Trouvé<br>Calculé | C% | 59,7<br>59,42 | N% | 4,56<br>4,33 | H% | 6,66<br>6,54 |

Exemple 6.

Ester méthylique de la (S)-N-((acétylthiométhyl)-2 oxo-1 phényl-3 propyl)-(S) alanine :

L'acide S(-) est couplé dans les conditons décrites pour l'obtention de l'exemple 2 avec le (S) alaninate de méthyle. Ce composé sera désigné ci-après, composé (6).

Rendement : 88 %

F : 83 ° C (Microscope)

$(\alpha)_D^{25}$ = - 70,9 ° (1,3, MeOH)

IR : 3300, 1755, 1695, 1650 (nujol)

RMN[1]H : (CDCl3) : 7,2 (s, 5H) ; 6,6 (m, 1H) ; 4,5 (m, 1H) ; 2,7 à 3,3 (m, 5H) ; 2,3 (s, 3H) ; 1,33 (d, J = 7,3 Hz, 3 H).

| Microanalyse : | | | | | | |
|---|---|---|---|---|---|---|
| Trouvé<br>Calculé | C% | 59,3<br>59,42 | N% | 4,42<br>4,33 | H% | 6,46<br>6,54 |

A/ - ETUDE BIOLOGIQUE.

Dosage de l'activité "Enképhalinasique" et de l'activité de l'angiotensine convertase (ACE) avec détermination de l'effet des inhibiteurs in vitro et chez la souris in vivo : inhibition des mêmes enzymes et protection du facteur auriculaire natriurétique.

Les résultats sont rassemblés dans les tableaux ci-dessous.

ACTIVITES INHIBITRICES DE L'ENKEPHALINASE ET DE L'ACE IN VITRO ET IN VIVO.

TABLEAU 1.

| COMPOSES | Enképhalinase | | ACE | |
|---|---|---|---|---|
| | In vitro(a) (IC50'nM) | In vivo(b) (DE50'mg/kg) | In vitro(c) (IC50'nM) | In vivo(d) (DE50'mg/kg) |
| (1) | 2 | 0,8 | 5.000 | inactif (>20) |
| (2) | 3 | 0,4 | 100 | 10 |
| (5) | 3 | 0,9 | 2000 | inactif (>20) |
| (6) | 1,2 | 0,3 | 12 | 4 |

.(a) Activité mesurée sur membranes de striatum selon Llorens et al., J. Neurochem, 1982, 39, 1081.

.(b) Activité mesurée dans le striatum de souris 30 mn après administration i.v. selon Llorens-Cortes et al., Eur. J. Pharmacol., 1985, 119, 183.

.(c) et (d) Activités mesurées sur membranes de striatum de souris in vitro ou 30 mn après administration i.v. selon Yang et Neff, J. Neurochem., 1972, 19, 2443.

ACTIVITES INHIBITRICES DE L'EHKEPHALINASE ET PROTECTION DU FACTEUR AURICULAIRE NATRIURETIQUE (ANF) DANS LE REIN IN VIVO

TABLEAU 2

| | Inhibition de l'enképhalinase (%) (a) | Augmentation de l'ANF (%) (b) |
|---|---|---|
| COMPOSE (1) | 15 ± 6 | inactif |
| COMPOSE (2) | 54 ± 6 | 33 ± 3 |

(a) Inhibition de liaison d'acétorphan $^{3}H$ à l'enképhalinase rénale mesurée selon S. de la Baume, M. Tuong, F. Brion, J.C. Schwartz (J.P.E.T. 1988, 247, 653) 90 minutes après administration orale des composés 1 ou 2 à la dose de 0,3 mg/kg.
(b) Augmentation du taux rénal d'ANF $^{125}I$, mesuré 2 mn après l'administration i.v. de $10^{6}$ cpm à des souris ayant reçu les composés 1 ou 2, 90 mn auparavant (0,3 mg/kg, par voie orale).
Le composé 2 (0,3 mg/kg, p.o.) induit dans le même test une augmentation de l'ANF $^{125}I$ rénale de 75 ± 7 % après 30 mn et de 20 ± 5 % après 180 mn.

B/ - ETUDE PHARMACOLOGIQUE

L'étude pharmacologique des produits décrits ci-dessus a permis de mettre en évidence un effet antalgique, psychotrope, antidiarrhéique, anti-acide gastrique, et un effet anti-inflammatoire dans la cholécystite expérimentale.

Les épreuves pharmacologiques réalisées ont été les suivantes:

I. TOXICITE AIGUE

La détermination de la mortalité chez la souris est observée à la suite de l'administration unique par voie intraveineuse de doses croissantes des composés à tester.

La DL 50, pour les composés étudiés, est supérieure à 100 mg/kg/i.v.

II. TOXICITE SUBAIGUE

Le composé (1) a été administré pendant 3 semaines à des souris à la dose orale de 2 g/kg/jour. Aucune modification de l'évolution pondérale et aucun signe de toxicité n'ont été présentés par les animaux par rapport aux témoins. Le poids des organes et leur examen antomo-pathologique après sacrifice des animaux n'ont montré aucune différence par rapport aux témoins solvant.

Par ailleurs, chez les animaux chez qui on procédait à une épreuve de réversibilité à l'arrêt du traitement, aucun signe de tolérance, ni d'accoutumance et aucun phénomène de sevrage n'ont été observés.

III - ACTIVITES ANALGESIQUES MESUREES SUR LE TEST DU SAUT DE LA PLAQUE A 55 °C APRES ADMINISTRATION A LA SOURIS

(Eddy et Leimbach, J. Pharmacol. Exp. Ther. 1953, 107 385)

TABLEAU 3

| | Temps d'adm. avant épreuve | Dose (mg/kg) | Voie | Délai de réponse (sec) |
|---|---|---|---|---|
| Témoins | | | | 50 ± 5 |
| Composé (1) | 1h | 30 | orale | 80 ± 6* |
| Composé (1) | 1h | 100 | orale | 140 ± 10* |
| Composé (2) | 1h | 30 | orale | 75 ± 5* |
| Composé (1) | 20′ | 5 | i.v. | 85 ± 5* |

*P < 0,01

L'absence d'effet inhibiteur sur l'ACE du composé 1 permet de l'administrer à dose élevée.

IV - ACTIVITE ANTIDIARRHEIQUE MESUREE CHEZ LE RAT TRAITE A L'HUILE DE RICIN.
(Neimegeers et al., Arzneim. Forsch., 1974, 24, 1622)

TABLEAU 4

| | Délai d'apparition des selles diarrhéiques (min) |
|---|---|
| Témoins | 80′ ± 5 |
| Composé (1) (20 mg/kg,p.o.) | 190′ ± 30* |
| Composé (2) (10 mg/kg,p.o.) | 160′ ± 25* |
| Les composés (1) et (2) selon l'invention ont été administrés 30 mn avant l'huile de ricin. | |

* p < 0,01

V - ACTIVITE MOTRICE COLIQUE

Chez le chien en période post-prandiale, le composé (1) potentialise l'augmentation de la motricité colique en réponse à l'alimentation.

TABLEAU 5

| Index moteur (évalué par jauge de contrainte). | | | | |
|---|---|---|---|---|
| Voie | Dose (mg/kg) | Avant repas | Après repas | |
| | | (-2/0h) | (O/+2h) | (+2/1Oh) |
| Témoin - | - | 6,1±1,5 | 8,8±1,9 | 11,2±2,5 |
| Composé (1) i.v. | 1 | 5,9±0,7 | 15,2±2,4* | 18,3±3,1* |
| Composé (1) p.o. | 10 | 6,4±0,8 | 17,4±2,7* | 21,5±4,6* |
| Composé (2) p.o. | 5 | 6,2±0,9 | 16,3±2,5* | 20,1±3,7* |
| Influence de l'administration préalable du composé par voie intraveineuse (IV) ou orale (PO) sur l'évolution de l'index moteur colique à la suite d'un repas standard chez le chien. | | | | |

* p < 0,01

VI - ACTIVITE IHHIBITRICE DE L'ACTIVITE ACIDE GASTRIQUE.

Chez le chat, la stimulation de la sécrétion acide gastrique induite par la Pentagastrine, le 2-désoxy glucose et l'histamine est significativement (p < 0,05) diminuée par l'administration des composés (1) et (2) à la dose de 0,5 : 1,5 et de 5 mg/kg/i.v.

VII - ACTIVITE ANTI-INFLAMMATOIRE DANS LA CHOLECYSTITE EXPERIMENTALE.

Chez le chat (Méthode de SVANVIK J et al. SURGERY, 1981, 90, 500), à la dose de 3 mg/kg/i.v. - le composé (1) diminue significativement l'hypersécrétion induite par l'inflammation de la vésicule biliaire.

TABLEAU 6

| Composés | Sécrétion nette d'eau (ml/h) chez les animaux avec cholécystites (n = 10) |
|---|---|
| Témoin | 0,85 ± 0,23 |
| Composé (1) | 0,21 ± 0,15* |

* p < 0,05

C/ ETUDE DE PHARMACOLOGIE CLINIQUE

Chez des volontaires sains (9 sujets males), on a mesuré les taux plasmatiques d'enképhalinase et du facteur auriculaire natriurétique (ANF) ainsi que la diurèse et la natriurèse après administration orale soit de placebo, soit de (S) acétorphan.

Par rapport à la séquence placebo, l'enképhalinase plasmatique est significativement inhibée (p<0,001) de 62 % et l'ANF plasmatique augmentée de 50 % (p<0,05) 1 h. après l'administration de 30 mg de (S) acétorphan alors que la diurèse et la natriurèse sont respectivement augmentées de 42 % et de 46 % (p<0,05) 2 à 8 h. après l'administration du produit.

Les résultats de ces études mettent en évidence la faible toxicité et les intéressantes propriétés inhibitrices de l'enképhalinase, notamment comme : antalgique, psychotrope, antidiarrhéique régulateur de la motricité colique, anti-acide gastrique, anti-inflammatoire au cours des cholécystites expérimentales, des dérivés de l'invention ; l'effet protecteur du facteur auriculaire natriurétique (ANF) entraîne une activité diurétique et natriurétique utile au cours de l'insuffisance cardiaque et hépato-rénale et de l'hypertension artérielle.

Ces propriétés les rendent utiles en médecine humaine et médecine vétérinaire. Les composés (1), (3), et (5), (R), sont utiles à la fois pour des indications centrales et périphériques, notamment comme antalgique et psychotrope et comme médicament de la colopathie fonctionnelle, du reflux gastro-oesophagien et de la cholécystite aiguë. Ils sont administrables à des doses unitaires même élevées, de préférence de 50 à 500 mg de principe actif, et à une posologie quotidienne, de préférence, variant de 50 mg à 1 g de principe actif.

Les composés (2), (4) et (6), c'est-à-dire (S), seront utiles préférablement pour les indications périphériques (colopathie fonctionnelle, reflux gastro-oesophagien et cholécystite aiguë) et surtout dans les indications cardiovasculaires pour lesquelles l'important effet protecteur de l'ANF est mis à profit dans le traitement de l'insuffisance cardiaque et hépatorénale et de l'hypertension artérielle à des doses unitaires faibles, de préférence, de 5 à 100 mg de principe actif, et des posologies quotidiennes variant, de préférence, de 5 à 200 mg de principe actif.

Les médicaments de l'invention sont administrables à l'homme par voie orale, parentérale ou rectale.

## Revendications

**1.** Composé énantiomère (R) ou (S) ayant la formule

(1)

$$CH_3 - CO - S - CH_2 - \underset{\displaystyle CH_2 - C_6H_5}{\underset{|}{CH}} - CO - NH - CH_2 - COO\ CH_2 - C_6H_5 \quad (R)$$

ou

(2)

$$CH_3 - CO - S - CH_2 - \underset{\displaystyle CH_2 - C_6H_5}{\underset{|}{CH}} - CO - NH - CH_2 - COO\ CH_2 - C_6H_5 \quad (S).$$

ou

(3)

$$CH_3 - CO - S - CH_2 - \underset{\displaystyle CH_2 - C_6H_5}{\underset{|}{CH}} - CO - NH - CH_2 - COO - CH_3 \quad (R)$$

ou

(4)

$$CH_3 - CO - S - CH_2 - \underset{\displaystyle CH_2 - C_6H_5}{\underset{|}{CH}} - CO - NH - CH_2 - COO - CH_3 \quad (S)$$

**2.** Procédé de préparation du composé R (+) selon la revendication 1, caractérisé en ce que l'on réalise le dédoublement de l'acide acétylthio-3 benzyl 2-propanoïque racémique, notamment par réaction avec l'éphédrine (+), l'on récupère le sel de l'énantiomorphe (+) obtenu, on libère l'acide énantiomorphe et on effectue le couplage avec l'ester correspondant de l'acide aminé.

**3.** Procédé de préparation du composé S (-) selon la revendication 1, caractérisé en ce que l'on réalise le dédoublement de l'acide acétylthio-3 benzyl 2-propanoïque racémique, notamment par réaction avec l'éphédrine (-), l'on récupère le sel de l'énantiomorphe (-) obtenu, on libère l'acide énantiomorphe et on effectue le couplage avec l'ester correspondant de l'acide aminé.

**4.** Procédé selon l'une des revendications 2 et 3, caractérisé en ce que l'on procède au couplage de l'ester correspondant de l'acide aminé avec l'acide énantiomorphe en présence de DCC comme agent de couplage et HOBT pour éviter la racémisation.

**5.** Procédé selon l'une des revendications 2 à 4, caractérisé en ce que l'on procède à la libération de l'acide énantiomorphe par action d'acide chlorhydrique.

**6.** Médicament caractérisé en ce qu'il comporte, comme principe actif, l'un des composés selon la revendication 1.

**7.** Médicaments selon la revendication 6, notamment médicaments antalgiques et psychotropes, médicament du syndrome du colon irritable,médicament du reflux gastro-oesophagien et médicament de la cholécystite aiguë, caractérisé en ce qu'il comporte , comme principe actif, un composé R (+).

**8.** Médicaments selon la revendication 7, caractérisé par une dose unitaire comprise entre 50 et 500 mg de principe actif, les doses administrables journellement pouvant varier entre 50 mg et 1 g.

9. Médicaments selon la revendication 6, notamment médicaments à indication cardio-vasculaire, y compris médicament pour le traitement de l'hypertension artérielie, médicament pour le traitement de l'insuffisance cardiaque et médicament pour le traitement de l'insuffisance hépato-rénale, caractérisé en ce qu'il comporte, comme principe actif, un composé S (-).

10. Médicament selon la revendication 9, caractérisé par une dose unitaire de 5 à 100 mg de principe actif, les doses administrables journellement étant comprises entre 5 et 200 mg.

11. Utilisation de l'un des composés R (+) selon la revendication 1 ou du composé ayant la formule suivante :

(5)

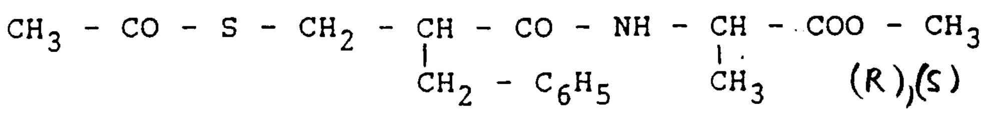

pour la préparation d'un médicament pour le traitement du syndrome du colon irritable, d'un médicament pour le traitement du reflux gastro-oesophagien ou d'un médicament pour le traitement de la cholécystite aiguë.

12. Utilisation selon la revendication 11, caractérisée en ce que l'on conditionne le composé en dose unitaire comprise entre 50 et 500 mg de principe actif, les doses administrables journellement pouvant varier entre 50 mg et 1 g.

13. Utilisation d'un composé S (-) selon la revendication 1 ou du composé ayant la formule suivante :

(6)

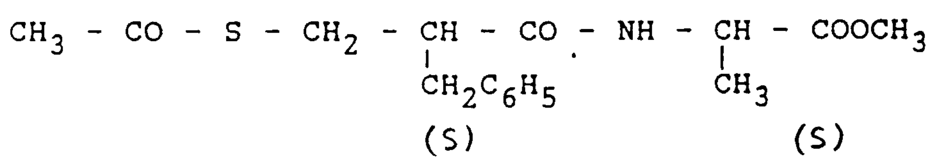

pour la préparation d'un médicament pour le traitement de l'hypertension artérielle, d'un médicament pour le traitement de l'insuffisance cardiaque ou d'un médicament pour le traitement de l'insuffisance hépato-rénale.

14. Utilisation selon la revendication 13, caractérisée en ce que l'on conditionne ledit composé en dose unitaire de 5 à 100 mg de principe actif, les doses administrables journellement étant comprises entre 5 et 200 mg.

## Claims

1. An (R) or (S) enantiomer compound having the formula

(1)

$$CH_3 - CO - S{-}CH_2 - \underset{\displaystyle CH_2 - C_6H_5\ (R)}{\underset{|}{CH}} - CO - NH - CH_2 - COO\ CH_2 - C_6H_5$$

or

(2)

$$CH_3 - CO - S - CH_2 - \underset{\displaystyle CH_2 - C_6H_5\ (S)}{\underset{|}{CH}} - CO - NH - CH_2 - COO\ CH_2 - C_6H_5$$

or

(3)

$$CH_3 - CO - S - CH_2 - \underset{\displaystyle CH_2 - C_6H_5\ \ (R)}{\underset{|}{CH}} - CO - NH - CH_2 - COO - CH_3$$

or

(4)

$$CH_3 - CO - S - CH_2 - \underset{\displaystyle CH_2C_6H_5\ \ (S)}{\underset{|}{CH}} - CO - NH - CH_2 - COO - CH_3$$

2. A process for preparing (R)-(+) compound according to claim 1, characterized in that the splitting of racemic 3-acetylthio-2-benzylpropanoic acid is effected notably by reacting with (+)-ephedrin, that the (+) enantiomorph salt obtained is recovered, that the enantiomorph acid is freed and that the coupling is effected with the corresponding amino-acid ester.

3. A process for preparing (S)-(-) compound according to claim 1, characterized in that the splitting of racemic 3-acetylthio-2-benzylpropanoic acid is effected notably by reaction with (-)-ephedrin, that the (-) enantiomorph salt obtained is recovered, that the enantiomorph acid is freed and that the coupling is effected with the corresponding amino-acid ester.

4. A process according to any of claims 2 and 3, characterized in that the coupling of the corresponding amino-acid ester is effected with the enantiomorph acid in the presence of DCC as coupling agent and HOBT to avoid racemisation.

5. A process according to any of claims 2 to 4, characterized in that the enantiomorph acid is freed by the action of hydrochloric acid.

6. A drug characterized in that it contains, as active principle, one of the compounds according to claim 1.

7. A drug according to claim 6, notably antalgic and psychotropic drugs, drug for irritable bowl syndrom, drug for gastro-oesophageal regurgitation and drug for acute cholecystitis, characterized in that it contains, as active principle, a (R)-(+) compound.

8. A drug according to claim 7, characterized by a unit dose which is between 50 and 500 mg of active principle, the daily administrable doses being able to vary between 50 mg and 1 g.

9. A drug according to claim 6, notably cardiovascular indicated drugs, including drug for the treatment of high blood pressure, drug for the treatment of cardiac insufficiency and drug for the treatment of hepatorenal insufficiency, characterized in that it contains, as active principle, a (S)-(-) compound.

**10.** A drug according to claim 9, characterized by a unit dose of 5 to 100 mg of active principle, the daily administrable doses being between 5 and 200 mg.

**11.** The use of one of (R)-(+)-compounds according to claim 1 or of the compound having the following formula :

(5)

$$CH_3 - CO - S - CH_2 - \underset{\displaystyle CH_2-C_6H_5}{\underset{|}{CH}} - CO - NH - \underset{\displaystyle CH_3}{\underset{|}{CH}} - COO - CH_3 \quad (R),\ (S)$$

for preparing a drug for the treatment of irritable bowl syndrom, a drug for the treatment of gastro-oesophageal regurgitation or a drug for the treatment of acute cholecystitis.

**12.** The use according to claim 11, characterized in that the compound is packaged in unit dose which is between 50 and 500 mg of active principle, the daily administrable doses being able to vary between 50 mg and 1 g.

**13.** The use of a (S)-(-) compound according to claim 1 or of the compound having the following formula :

(6)

$$CH_3 - CO - S - CH_2 - \underset{\displaystyle CH_2 - C_6H_5}{\underset{|}{CH}} - CO - NH - \underset{\displaystyle CH_3}{\underset{|}{CH}} - COOCH_3 \quad (S),\ (S)$$

for preparing a drug for the treatment of high blood pressure, a drug for the treatment of cardiac insuffiency or a drug for hepato-renal insufficiency.

**14.** The use according to claim 13, characterized in that said compound is packaged in unit dose from 5 to 100 mg of active principle, the daily administrable doses being between 5 and 200 mg.

## Patentansprüche

**1.** (R)- oder (S)-enantiomere Verbindungen der Formel

(1)

$$CH_3 - CO - S - CH_2 - CH - CO - NH - CH_2 - COO\ CH_2 - C_6H_5$$

|

$CH_2 - C_6H_5$ (R)

oder

(2)

$$CH_3 - CO - S - CH_2 - CH - CO - NH - CH_2 - COO\ CH_2 - C_6H_5$$

|

$CH_2 - C_6H_5$ (S)

oder

(3)

$$CH_3 - CO - S - CH_2 - CH - CO - NH - CH_2 - COO - CH_3$$

|

$CH_2 - C_6H_5$ (R)

oder

(4)

$$CH_3 - CO - S - CH_2 - CH - CO - NH - CH_2 - COO - CH_3$$

|

$CH_2 - C_6H_5$ (S)

**2.** Verfahren zur Herstellung der R-(+)-Verbindung gemäß Anspruch 1, dadurch gekennzeichnet, daß man die Spaltung des 3-Acetylthio-2-benzyl-propansäureracemats insbesondere mittels Reaktion mit (+)-Ephedrin durchführt, man das so erhaltene (+)- enantiomorphe Salz gewinnt, die enantiomorphe Säure freisetzt und mit dem korrespondierenden Aminosäureester koppelt.

**3.** Verfahren zur Herstellung der S-(-)-Verbindung gemäß Anspruch, 1 dadurch gekennzeichnet, daß man die Aufspaltung des 3-Acetylthio-2-benzyl-propansäureracemats insbesondere durch Reaktion mit (-)-Ephedrin durchführt, das so erhaltene (-)- enantiomorphe Salz gewinnt, die enantiomorphe Säure freisetzt und die Kupplung mit dem korrespondierenden Aminosäureester durchführt.

**4.** Verfahren nach einem der Ansprüche 2 und 3, dadurch gekennzeichnet, daß man die Kupplung mit dem korrespondierenden Aminosäureester mit der enantiomorphen Säure in Gegenwart von DCC als Kopplungsagens und HOBT durchführt, um eine Racemisierung zu vermeiden.

**5.** Verfahren nach einem der Ansprüche 2 bis 4, dadurch gekennzeichnet, daß man die Freisetzung der enantiomorphen Säure mit Salzsäure durchführt.

**6.** Arzneimittel, dadurch gekennzeichnet, daß es als Wirkstoff eine der Verbindungen nach Anspruch 1 enthält.

**7.** Arzneimittel nach Anspruch 6, insbesondere antalgetische und psychotrope Arzneimittel, Arzneimittel für das Darmreizungssyndrom, Arzneimittel für die Reflux-Ösophagitis und Arzneimittel für die akute Cholecystitis, dadurch gekennzeichnet, daß es als Wirkstoff eine R-(+)-Verbindung enthält.

**8.** Arzneimittel nach Anspruch 7, gekennzeichnet durch eine Einzeldosis zwischen 50 und 500 mg aktiven Wirkstoffes, wobei die zu verabreichenden Tagesdosen zwischen 50 mg und 1 g variieren können.

**9.** Arzneimittel nach Anspruch 6, insbesondere Arzneimittel für cardiovaskuläre Indikationen, einschließlich Arzneimittel zur Behandlung des arteriellen Bluthochdruckes, Arzneimittel zur Behandlung der Herzinsuffizienz und Arzneimittel zur Behandlung der hepatorenalen Insuffizienz, dadurch gekennzeichnet, daß es als Wirkstoff eine S-(-)-Verbindung enthält.

**10.** Arzneimittel nach Anspruch 9, gekennzeichnet durch eine Einzeldosis von 5 bis 100 mg aktiven Wirkstoffes, wobei die zu verabreichenden Tagesdosen zwischen 5 und 200 mg betragen.

**11.** Verwendung einer R-(+)-Verbindung nach Anspruch 1 oder einer Verbindung der folgenden Formel

(5)

$CH_3 - CO - S - CH_2 - CH - CO - NH - CH - COO - CH_3$

$\qquad\qquad\qquad\qquad\qquad\qquad |\qquad\qquad\qquad\quad |$

$\qquad\qquad\qquad\qquad\qquad CH_2 - C_6H_5 \qquad CH_3 \qquad (R),(S)$

zur Herstellung eines Medikamentes zur Behandlung des Darmreizungssyndroms, eines Medikamentes zur Behandlung der Reflux-Ösophagitis oder eines Medikamentes zur Behandlung der akuten Cholecystitis.

**12.** Verwendung nach Anspruch 11, dadurch gekennzeichnet, daß man eine Einzeldosis mit 50 bis 500 mg aktiven Wirkstoff zubereitet, wobei die zu verabreichenden Tagesdosen zwischen 50 mg und 1 g variieren können.

**13.** Verwendung einer S-(-)-Verbindung nach Anspruch 1 oder einer Verbindung der folgenden Formel

(6)

$CH_3 - CO - S - CH_2 - CH - CO - NH - CH - COOCH_3$

$\qquad\qquad\qquad\qquad\qquad\qquad |\qquad\qquad\qquad\quad |$

$\qquad\qquad\qquad\qquad\qquad CH_2C_6H_5 \qquad\qquad CH_3$

(S),(S)

zur Herstellung eines Arzneimittels zur Behandlung des arteriellen Bluthochdruckes, eines Medikamentes zur Behandlung der Herzinsuffizienz oder eines Medikamentes zur Behandlung der hepatorenalen Insuffizienz.

**14.** Verwendung nach Anspruch 13, dadurch gekennzeichnet, daß man die Verbindung in Einzeldosen von 5 bis 100 mg Wirkstoff zubereitet, wobei die zu verabreichenden Tagesdosen zwischen 5 und 200 mg betragen.